Europäisches Patentamt

European Patent Office

Office européen des brevets

(11) Publication number : **0 508 783 A1**

(12) # EUROPEAN PATENT APPLICATION

(21) Application number : 92303175.1

(22) Date of filing : 09.04.92

(51) Int. Cl.$^5$ : **C12N 7/04,** C12N 15/01,
     A61K 39/13

(30) Priority : **10.04.91 GB 9107563**

(43) Date of publication of application :
     **14.10.92 Bulletin 92/42**

(84) Designated Contracting States :
     **AT BE CH DE DK ES FR GB GR IT LI LU MC NL PT SE**

(71) Applicant : **BRITISH TECHNOLOGY GROUP LTD**
     **101 Newington Causeway**
     **London SE1 6BU (GB)**

(72) Inventor : **Macadam, Andrew Joseph, Nat. Inst. for Biological**
     **Standards and Control, Blanche Lane, South Mimms**
     **Potters Bar, Herts EN6 3QG (GB)**
     Inventor : **Minor, Philip David, Nat. Inst. for Biological**
     **Standards and Control, Blanche Lane, South Mimms**
     **Potters Bar, Herts EN6 3QG (GB)**
     Inventor : **Stone, David Michael, Dept. of Microbiology**
     **University of Reading**
     **Whiteknights, Reading RG6 2AJ (GB)**
     Inventor : **Almond, Jeffrey William, Dept. of Microbiology**
     **University of Reading**
     **Whiteknights, Reading RG6 2AJ (GB)**

(74) Representative : **Woods, Geoffrey Corlett et al**
     **J.A. KEMP & CO. 14 South Square Gray's Inn**
     **London WC1R 5LX (GB)**

(54) Attenuated poliovirus and vaccines thereof.

(57)  A poliovirus which is either a type 3 poliovirus having amino acid residues valine and alanine at positions 54 and 114 respectively of the VP1 capsid protein or is a type 1 or type 2 poliovirus having equivalent amino acid residues at corresponding positions.

EP 0 508 783 A1

This invention relates to the construction of vaccines against polioviruses, by the introduction of defined mutations into their genomes.

At the present time, the only vaccines routinely used againt enterovirus and rhinovirus infections are those against poliomyelitis. Of these the live attenuated vaccines developed by Sabin in the 1950's have found greatest use throughout the world. Vaccine strains derived from each of the three poliovirus serotypes (P1, P2 and P3) were prepared by passage of wild type viruses in cell cultures and whole animals until attenuated strains were obtained. These attenuated viruses are substantially less able to cause poliomyelitis in humans than the original wild type strains. They are administered orally and replicate in the gut to induce a protective immune response.

Although these vaccines are generally regarded as safe, their use is associated with a low level of paralysis in vaccinees. This is most often associated with type 2 and type 3 serotypes and rarely, if ever, with type 1. There is therefore a requirement for improved type 2 and type 3 vaccines which would be comparable in safety to the excellent type 1 strain.

The Sabin vaccine strains were developed by essentially empirical procedures. The genetic basis of their attenuation is not properly understood. Over the past few years, however, scientists have employed a number of molecular biological techniques in an attempt to elucidate the mechanism by which the neurovirulence of these vaccine strains is reduced. Most of the work has concentrated on serotypes 1 and 3. For both of these the complete nucleotide sequences of the vaccine strains have been compared with those of their neurovirulent progenitors.

In the case of poliovirus type 1, the vaccine strain differs from its progenitor at 57 positions in the 7441 base genome (Nomoto et al, 1982, Proc Natl Acad Sci USA 79 : 5793-5797). All of these are simple point mutations and 21 of them give rise to amino acid changes in virus coded proteins. Although several mutations are thought to contribute to the attenuation phenotype of the vaccine strain, direct evidence has been presented that the mutation of A to G at position 480 in the 5′ non-coding region of the genome has a marked attenuating effect on the virus (Nomoto et al, 1987, UCLA Symp Mol Cell Biol, New Series, Vol 54 (Eds M A Brinton and R R Rueckert), 437-452, New York : Alan R Liss Inc).

Analogous studies on poliovirus type 3 reveal just 10 nucleotide sequence differences in the 7432 base genome between the vaccine and its progenitor strain (Stanway et al, 1984, Proc Natl Acad Sci USA 81 : 1539-1543). Just three of these give rise to amino acid substitutions in virus encoded proteins. The construction of defined recombinants between the vaccine and its progenitor strain has allowed the identification of the mutations which contribute to the attenuation phenotype. One of these is at position 2034 and causes a serine to phenylalanine change in virus protein VP3.

The other mutation of interest is C to U at position 472 in the 5′ non-coding region of the genome. This latter mutation has been observed to revert to the wild type C rapidly upon replication of the virus in the human gut (Evans et al, 1985, Nature 314 : 548-550). This reversion is associated with an increase in neurovirulence. C at position 472 has also been shown to be essential for growth of a mouse/human polio recombinant virus in the mouse brain (La Monica et al, 1986, J Virol 57 : 515-525). Recently, we have observed that at 481 in poliovirus type 2 A changes to G in an analogous fashion upon replication of the type 2 vaccine in the gut of vaccinees.

In EP-A-0383433 attenuated enteroviruses, particularly polioviruses, and rhinoviruses are described which have a reversed base pairing in the part, or in a part corresponding to the part, of the 5′ non-coding region of the genome of poliovirus type 3 Leon strain shown below:

```
        471          477          483

    ... U C C .... C C A U G G A ....

    ... A G G .... G G U G C C U ....

        538          534          528
```

EP-A-0383433 indicates that a suitable attenuated poliovirus of type 1 or type 2 has the bases G and C at positions 469 and 534 respectively and that a suitable attenuated poliovirus of type 3 has the bases G and C at positions 472 and 537 respectively.

We have now obtained a type 3 poliovirus in which the amino acid residues at positions 54 and 114 of the VP1 capsid protein are valine and alanine respectively. The virus particles are relatively thermostable. They are more resistant to elevated temperatures than the Sabin strain of type 3 poliovirus which has amino acid residues alanine and valine at positions 54 and 114 of the VP1 protein. Further, the growth of the virus is temperature sensitive in a similar fashion to the type 3 Sabin strain.

Our findings can be extrapolated to all polioviruses. Amino acid substitutions at positions of the VP1 capsid proteins of type 1 and type 2 polioviruses corresponding to positions 54 and 114 of the VP1 protein of poliovirus-type 3 Leon strain can also lead to thermostability and attenuation. There is a relatively high degree of homology between the genomic RNA, and between amino acid sequences of proteins, of the three types of poliovirus. The positions of amino acid residues of a capsid protein of a strain of poliovirus of type 1 or type 2 which correspond to positions of amino acid residues of a capsid protein of poliovirus type 3, for example of poliovirus type 3 Leon strain, can be determined by lining up the amino acid sequences of the strains. This has been done for the Sabin strains of each type of poliovirus in Toyoda et al, 1984, J. Mol. Biol. 174:561-585.

Accordingly the invention provides a poliovirus which is either a type 3 poliovirus having amino acid residues valine and alanine at positions 54 and 114 respectively of the VP1 capsid protein or is a type 1 or type 2 poliovirus having equivalent amino acid residues at corresponding positions.

The positions of the VP1 capsid protein of type 1 poliovirus and of type 2 poliovirus which correspond to positions 54 and 114 of the VP1 capsid protein of type 3 poliovirus are positions 56 and 116. The normal amino acid residues at these positions 56 and 116 are replaced in accordance with the invention by amino acid residues which result in a type 1 or type 2 poliovirus which is thermostable and whose growth is temperature sensitive in substantially the same way as a type 3 poliovirus according to the invention. Appropriate amino acid substitutions may be made accordingly. Indeed, the amino acid residues at positions 56 and 116 of the VP1 protein of a type 1 or type 2 poliovirus may be valine and alanine respectively.

Further amino acid substitutions may be made. A useful poliovirus according to the invention has a methionine amino acid residue at position 215 of the VP2 capsid protein in the case of a type 3 poliovirus or an equivalent amino acid residue at the corresponding position in the case of a type 1 or type 2 poliovirus. The corresponding position is position 215 for type 1 poliovirus and position 214 for type 2 poliovirus. An appropriate amino acid substitution for a type 1 or type 2 poliovirus may again be selected which results in a poliovirus which is thermostable and whose growth is temperature sensitive in substantially the same way as a type 3 poliovirus according to the invention incorporating a methionine residue at VP2 residue 214. An appropriate amino acid for VP2 position 215 of a type 1 poliovirus or for VP2 position 214 of a type 2 poliovirus may therefore be methionine.

The poliovirus may be a poliovirus in which the 5′ non-coding region of the genome is:

(a) the 5′ non-coding region of poliovirus type 3 Leon strain modified by the provision of bases uracil and adenine at positions 472 and 537 thereof respectively, or

(b) the 5′ non-coding region of another poliovirus modified by the provision of bases uracil and adenine at respective positions corresponding to positions 472 and 537 of the 5′ non-coding region of poliovirus type 3 Leon strain.

The attenuated poliovirus may be a type 1, type 2 or type 3 poliovirus. Types 2 and 3 are preferred. For types 1 and 2, positions 469 and 534 correspond to positions 472 and 537 respectively of poliovirus type 3. The numbering system of the positions of the 5′ non-coding region of poliovirus type 3, in particular of poliovirus type 3 Leon strain, is the numbering system employed in the Stanway et al paper above.

Any strain of poliovirus may be attenuated according to the present invention. An attenuated strain of type 3 poliovirus has the bases U and A at positions 472 and 537 respectively of the genome. An attenuated strain of type 1 or type 2 poliovirus has the bases U and A at the positions of the genome which correspond to positions 472 and 537 of the genome of poliovirus type 3 Leon strain.

The amino acid sequence of the proteins of a poliovirus according to the present invention may be identical, apart from the specific amino acid residues noted above, to that of the proteins of any poliovirus, for example a vaccine strain such as a Sabin strain or a wild-type strain such as type 1 Mahoney strain or type 3 Leon strain. Similarly, the genome of a poliovirus according to the invention may be identical, apart from the changes required to cater for the present invention, to that of any poliovirus, for example a vaccine strain such as a Sabin strain or a wild-type strain such as type 1 Mahoney strain or type 3 Leon strain.

An attenuated virus according to the invention is prepared by a process comprising:

(i) sub-cloning a portion of a cDNA of a poliovirus, which portion includes the coding sequence for the amino acid residues at positions 54 and 114 of the VP1 capsid protein in the case where the poliovirus is a type 3 poliovirus or at corresponding positions in the case where the poliovirus is a type 1 or type 2 poliovirus;

(ii) mutating the said sub-cloned portion at the codons for the said positions 54 and 114 or for the said corresponding positions so that the codons for the said positions 54 and 114 encode the amino acid residues valine and alanine respectively or the codons for the said corresponding positions encode respective amino acid residues equivalent thereto;

(iii) reintroducing the thus mutated portion or a fragment thereof which includes the mutated positions into the complete cDNA from which the portion was derived; and

(iv) obtaining live virus from the cDNA thus obtained.

Mutations as required for other embodiments of the invention may be made in analogous fashion. A mutation can be introduced into a strain of a poliovirus, for example a non-virulent strain such as a vaccine strain or a virulent strain such as a wild-type strain, by site-directed mutagenesis. The poliovirus may be a Sabin, type 3 Leon or type 1 Mahoney strain. Mutation may be achieved beginning with sub-cloning the appropriate region from an infectious DNA copy of the genome of any of the virus strain, for example a vaccine strain or its progenitor, into the single strand DNA of a bacteriophage such as M13. The desired mutation is then introduced into this sub-cloned cDNA using the technique of oligonucleotide directed mutagenesis.

After the introduction of mutation, the modified sub-cloned cDNAs are reintroduced into the complete cDNA from which they are derived and, for virulence testing in mice, into a cDNA derived from a murine poliovirus derivative known to cause a poliomyelitis type disease in mice (La Monica et al, 1986). Live virus is recovered from the mutated full length cDNA by production of a positive sense RNA typically using a T7 promoter to direct transcription in vitro (Van der Werf et al, 1986, Proc NatlAcad Sci, USA 83 : 2330-2334). The recovered RNA may be applied to tissue cultures using standard techniques (Koch, 1973, Curr Top Microbiol Immunol 61 : 89-138). After 4-6 days incubation virus can be recovered from the supernatant of the tissue culture. The level of neurovirulence of the modified virus may then be compared with that of the unmodified virus using a standard LD50 test in mice (La Monica et al, 1986) or the WHO approved vaccine safety test in monkeys (WHO Tech Rep Ser 687 : 107-175, 1983).

The attenuated viruses can be used as vaccines. They may therefore be formulated as pharmaceutical compositions further comprising a pharmaceutically acceptable carrier or diluent. Any carrier or diluent conventionally used in vaccine preparations may be employed. For example, the presently used live attenuated poliovirus strains are stabilised in a solution of 1 molar $MgCl_2$ and administered as a mixture of the three serotypes.

The attenuated viruses can therefore be used to prevent an infection attributable to a poliovirus in a human patient. For this purpose, they may be administered orally, as a nasal spray, or parenterally, for example by subcutaneous or intramuscular injection. A dose corresponding to the amount administered for a conventional live virus vaccine, such as up to $10^6$ $TCID_{50}$ for a Sabin vaccine strain in the case of poliovirus, may be administered.

The following Example illustrates the invention.


EXAMPLE


Derivation and characterisation of isolate H143


Isolate H143 is a derivative of the Sabin strain of type 3 poliovirus (P3/Sabin) with differences at 4 nucleotide positions (1592, 2632, 2637 and 2817) leading to amino acid substitutions at VP2-215 (Leu-Met), VP1-054 (Ala-Val) and VP1-114 (Val-Ala) with respect to P3/Sabin. This virus is temperature-sensitive for growth (and hence presumed to be attenuated) and, at the same time relatively thermostable.

Two of the three changes (at VP2-215 and VP1-054) were introduced by site-directed mutagenesis. Briefly, a SmaI/SstI fragment of cloned P3/Sabin from nucleotide (nt) 752 to nt 2768 was subcloned into M13 mp 18 and mutagenised using the Mutagene kit (Biorad) and the following primers: 5′AATGGCCATAGCATTCA3′ and 5′CGGATGGTACCAGTGGATTGG3′.

The mutated fragment was then re-integrated into the full length infectious P3/Sabin cDNA cloned into pBR322, under the control of a T7 promoter. Recombinant DNA was cleaved with SalI prior to RNA transcription and transfection of HEp2C cells. The recovered virus (H53) was sequenced through the mutated regions to verify its genotype.

Growth of H53 in Hep cells was assayed (Macadam et al, Virology, 172, 408). Plaque formation was found to be sensitive to temperatures below 31°C. Isolate H143 was selected from H53 by plaque-assay at 30°C, at which temperature titres were reduce 100-fold. The sequence of H143 was found to be the same as that of H53 at nucleotides 1592, 2632 and 2637 but a mutation was identified at 2817 (U→C) leading to a Val→Ala substitution at VP1-114. In all, about 700 nucleotides were sequenced by primer extension of the genomic RNA (Macadam et al, Virology, 172, 408) out of approximately 2,600 nucleotides that comprise the P1 coding region.

Growth of H143 was found to be as sensitive to elevated temperatures as that of P3/Sabin. However H143 virions were found to be considerably more thermostable than those of P3/Sabin. Preparations of H143 virus were grown in HEp2C cells and incubated at temperature of 4°, 20°, 37°, 45°C for different periods of time, before residual infectivity was assayed by conventional $TCID_{50}$ assay on HEp2C cells. Thermolability assays of H53, H143, P3/Sabin and two site-directed mutants with single changes at VP2-215 or VP1-054 showed that while all three mutations affected temperature-sensitivity for growth only those at VP1-054 and VP1-114 affected thermal stability. The thermal stability of H143 was compared with that of Sabin type 3 strain. Virus pools of the Sabin type 3 vaccine strain (designated SP6) and the variant H143 were grown in the human epithelial cell derived continuous cell line HEp2C in Minimal Essential Medium using standard protocols. Viruses were incubated in 0.5 ml aliquots in vials for the stated times at room temperature, 37°C, 40°C and 45°C. Three vials were taken for each temperature/time point and placed immediately at -20°C. Virus was assayed in microtitre plates using ten-fold dilution steps in eight replicate wells per dilution with HEp2C cells as the cell substrate by standard protocols. The incubated samples were assayed in parallel with three vials which had been maintained at -20°C throughout and with a reference virus preparation. The mean titre of the replicate vials was determined and the loss of titre on incubation compared to the unheated samples was calculated.

The results are summarised in the table.

$LOG_{10}$ difference* between unheated and heated

| | | | SP6 | H143 |
|---|---|---|---|---|
| 45° | 15 min | | 4.1 | 2.2 |
| | 30 min | > | 5.0 | 2.1 |
| | 45 min | > | 5.0 | 2.4 |
| 40° | 1 hr | | 0.6 | 0.5** |
| | 2 hr | | 1.8 | 0.4 |
| | 3 hr | | 2.1 | 0.2 |
| 37° | 1 d | | 2.4 | 1.6 |
| | 2 d | | 4.8 | 3.0 |
| | 3 d | | 4.9 | 3.6 |
| r t | 7 d | | 0.7 | 0.2 |
| | 14 d | | 0.6 | 0.5 |
| | 21 d | | 2.1 | 0.9 |

\*    mean of 3 samples
\*\*   no signficant loss over period of heating (significant loss = >0.6 log)

At all four temperatures studied H143 lost less infectivity than SP6 for a given incubation period. Under the conditions used in this study H143 was more thermostable than the parental Sabin strain.

## Claims

1. A poliovirus which is either a type 3 poliovirus having amino acid residues valine and alanine at positions 54 and 114 respectively of the VP1 capsid protein or is a type 1 or type 2 poliovirus having equivalent amino acid residues at corresponding positions.

2. A poliovirus according to claim 1, which in the case of a type 3 poliovirus has a methionine amino acid residue at position 215 of the VP2 capsid protein or in the case of a type 1 or type 2 poliovirus has an equivalent amino acid residue at the corresponding position.

3. A poliovirus according to claim 1 or 2, in which the 5′ non-coding region of the genome is
   (a) the 5′ non-coding region of poliovirus type 3 Leon strain modified by the provision of bases uracil and adenine at positions 472 and 537 thereof respectively, or
   (b) the 5′ non-coding region of another poliovirus modified by the provision of bases uracil and adenine at respective positions corresponding to positions 472 and 537 of the 5′ non-coding region of poliovirus type 3 Leon strain.

4. A poliovirus according to any one of the preceding claims, which is a type 1 poliovirus.

5. A poliovirus according to any one of claims 1 to 3, which is a type 2 poliovirus.

6. A poliovirus according to any one of claims 1 to 3, which is a type 3 poliovirus.

7. A process for the preparation of a poliovirus as defined in claim 1, which process comprises:
(i) sub-cloning a portion of a cDNA of a poliovirus, which portion includes the coding sequence for the amino acid residues at positions 54 and 114 of the VP1 capsid protein in the case where the poliovirus is a type 3 poliovirus or at corresponding positions in the case where the poliovirus is a type 1 or type 2 poliovirus;
(ii) mutating the said sub-cloned portion at the codons for the said positions 54 and 114 or for the said corresponding positions so that the codons for the said positions 54 and 114 encode the amino acid residues valine and alanine respectively or the codons for the said corresponding positions encode respective amino acid residues equivalent thereto;
(iii) reintroducing the thus mutated portion or a fragment thereof which includes the mutated positions into the complete cDNA from which the portion was derived; and
(iv) obtaining live virus from the cDNA thus obtained.

8. A pharmaceutical composition comprising a pharmaceutically acceptable carrier or diluent and a poliovirus as claimed in any one of claims 1 to 6.

## Claims for the following Contracting States : ES, GR

1. A process for the preparation of a poliovirus which is either a type 3 poliovirus having amino acid residues valine and alanine at positions 54 and 114 respectively of the VP1 capsid protein or is a type 1 or type 2 poliovirus having equivalent amino acid residues at corresponding positions, which process comprises:
(i) sub-cloning a portion of a cDNA of a poliovirus, which portion includes the coding sequence for the amino acid residues at positions 54 and 114 of the VP1 capsid protein in the case where the poliovirus is a type 3 poliovirus or at corresponding positions in the case where the poliovirus is a type 1 or type 2 poliovirus;
(ii) mutating the said sub-cloned portion at the codons for the said positions 54 and 114 or for the said corresponding positions so that the codons for the said positions 54 and 114 encode the amino acid residues valine and alanine respectively or the codons for the said corresponding positions encode respective amino acid residues equivalent thereto;
(iii) reintroducing the thus mutated portion or a fragment thereof which includes the mutated positions into the complete cDNA from which the portion was derived; and
(iv) obtaining live virus from the cDNA thus obtained.

2. A process according to claim 1, in which the poliovirus in the case of a type 3 poliovirus has a methionine amino acid residue at position 215 of the VP2 capsid protein or in the case of a type 1 or type 2 poliovirus has an equivalent amino acid residue at the corresponding position.

3. A process according to claim 1 or 2, in which in the poliovirus the 5′ non-coding region of the genome is
(a) the 5′ non-coding region of poliovirus type 3 Leon strain modified by the provision of bases uracil and adenine at positions 472 and 537 thereof respectively, or
(b) the 5′ non-coding region of another poliovirus modified by the provision of bases uracil and adenine at respective positions corresponding to positions 472 and 537 of the 5′ non-coding region of poliovirus type 3 Leon strain.

4. A process according to any one of the preceding claims, in which the poliovirus is a type 1 poliovirus.

5. A process according to any one of claims 1 to 3, in which the poliovirus is a type 2 poliovirus.

6. A process according to any one of claims 1 to 3, in which the poliovirus is a type 3 poliovirus.

7. A process for preparing a pharmaceutical composition comprising admixing a pharmaceutically acceptable carrier or diluent and a poliovirus produced by the process of any one of claims 1 to 6.

European Patent
Office

**EUROPEAN SEARCH REPORT**

Application Number

EP 92 30 3175

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF GENERAL VIROLOGY vol. 69, 1988, COLCHERTER, GB pages 1091 - 1096; P.D.MINOR ET AL: 'The effect of sequences in the 5' non-coding region on the replication of polioviruses in the human gut' * the whole document * | 1-8 | C12N7/04 C12N15/01 A61K39/13 |
| A | EP-A-0 323 900 (J.W. ALMOND) * the whole document * | 1-8 | |
| A | EP-A-0 383 434 (NATIONAL RESEARCH DEVELOPMENT CORPORATION) * the whole document * | 1-8 | |
| A | EP-A-0 325 768 (AMERICAN CYANAMIC CO.) * the whole document * | 1-8 | |
| A,D | EP-A-0 383 433 (NATIONAL RESEARCH CORP.) * the whole document * | 1-8 | |
| A,D | NATURE vol. 314, 11 April 1985, LONDON, GB pages 548 - 550; D.M.A. EVANS ET AL: 'increased neovirulence associated with a single nucleotide change in a noncoding region of the sabin type 3 polyovaccine genome' * the whole document * | 1-8 | TECHNICAL FIELDS SEARCHED (Int. Cl.5) A61K C12N C07K |
| A,D | PROCEEDINGS OF THE NATIONAL ACADEMY OF SCIENCES OF USA. vol. 81, March 1984, WASHINGTON US pages 1539 - 1543; G. STANWAY ET AL: 'comparison of the complete nucleotide sequences of the genomes of the neovirulent poliovirus p3/leon/37 and its attenuated sabin vaccine derivative p3/leon/12a1b' * the whole document * | 1-8 | |

The present search report has been drawn up for all claims

| Place of search | Date of completion of the search | Examiner |
|---|---|---|
| THE HAGUE | 22 JUNE 1992 | FERNANDEZ Y BRA F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

## EUROPEAN SEARCH REPORT

Application Number

EP 92 30 3175
Page 2

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | --- <br> JOURNAL OF MOLECULAR BIOLOGY <br> vol. 124, 1984, LONDON <br> pages 263 - 270; <br> H. TOYODA ET AL: 'complete nucleotide sequences of all three poliovirus serotype genomes.Implication for genetic relationship, gene function and antigenic determinants' <br> * the whole document * <br> --- | 1-8 | |
| A | BIOLOGICAL ABSTRACTS,vol 88, no 7, 1989, page AB-392, abstract no. 73356, biological abstracts inc., Philadelphia, PA, US; M. Skinner et al : "New model for the secondary structure of the 5' non-coding RNA of poliovirus is supported by biochemical and genetic data that also show that RNA secondary structure is important in neovirulence" &J. MOL. BIOL.207(2):379-392. 1989 <br> *abstract* <br> --- | 1-8 | |
| A | CHEMICAL ABSTRACTS, vol. 109, no. 25, 19 December 1988, Columbus, Ohio, US; abstract no. 228104, J.W.ALMOND ET AL: 'studies on the genetic basis of attenuation of the sabin type 3 poliovirus vaccine' <br> page 614 ; <br> & BIOCHEM. SOC. SYMP. 1986 (publ. 1987) 53, 85-90 <br> * abstract * <br> --- <br> -/-- | 1-8 | TECHNICAL FIELDS SEARCHED (Int. Cl.5) |

The present search report has been drawn up for all claims

| Place of search | Date of completion of the search | Examiner |
|---|---|---|
| THE HAGUE | 22 JUNE 1992 | FERNANDEZ Y BRA F. |

# EUROPEAN SEARCH REPORT

**European Patent Office**

**Application Number**

EP 92 30 3175
Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 114, no. 13, 1 April 1991, Columbus, Ohio, US; abstract no. 118401, R.REN ET AL: 'identification of two determinants that attenuate vaccine-related type 2 poliovirus' page 414 ; & J. VIROL. 1991 65(3), 1377-1382 * abstract * | 1-8 | |
| A,D | PROCEEDINGS OF THE NATIONAL ACADEMY OF SCIENCES OF USA. vol. 79, October 1982, WASHINGTON US pages 5793 - 5797; A.NOMOTO ET AL: 'complete nucleotide sequence of the attenuated poliovirus sabin 1 strain genome' * the whole document * | 1-8 | |
| | | | **TECHNICAL FIELDS SEARCHED (Int. Cl.5 )** |

The present search report has been drawn up for all claims

| Place of search | Date of completion of the search | Examiner |
|---|---|---|
| THE HAGUE | 22 JUNE 1992 | FERNANDEZ Y BRA F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)